# EUROPEAN PATENT APPLICATION

(11) **EP 3 493 358 A1**
(43) Date of publication of application: **05.06.2019**
(21) Application number: 18208909.4
(22) Date of filing: 28.11.2018
(51) Int. Cl.: H02J 9/06, A61B 6/00

(54) **SWITCHING PANEL**

(30) Priority: 30.11.2017 GB 201719919
(71) Applicant: TClarke Contracting Limited, London EC2Y 9AE (GB)
(72) Inventor: THOMPSON, Nigel Spencer, Great Leighs, Essex CM3 1NS (GB)
(74) Representative: Jaeger, Michael David

(57) **Abstract**

The present invention relates to systems (100) and methods for maintaining a power supply, comprising: monitoring a first power supply (125) and, upon detecting a decrease in the power received from the first power supply (125), sending a signal which causes a power demand device (140) to switch into a low power mode, and supplying power to the power demand device (140) from a second power supply (155).

## Description

The present invention relates to systems, methods and apparatuses for supplying power. In particular, the present invention relates to a switching panel, a system and a method of operating the switching panel for supplying uninterrupted power in specialist electrical safety environments, such as in healthcare settings.

### Background to the Invention

There are many specialist electrical safety environments which place exacting constraints on power supply systems. Exemplary environments include oil and gas extraction platforms and hospitals. These environments have restrictive electrical standards which place strict limits on electrical safety, including limits on line to line impedance and earth leakage.

In hospital environments, these restrictive limits are generally focused on ensuring patient safety. Consequently, these limits must be taken into careful consideration during the process of designing electrically powered installations for hospitals in a manner which may not be required for other venues. Hospital environments typically have two or more separate electrical circuits, including a *"low-risk circuit"* and a *"high-risk circuit".* The low-risk circuit is used to power devices in low-risk areas, such as waiting rooms, low-risk wards, staff rooms and the like. The high-risk circuit is used to power devices in high-risk areas, including surgical suites, imaging suites and high-dependency wards. Electrical devices, and the wiring to these devices, in such high-risk areas must conform to standards such as EN 60601 (a European Standard for Medical Electrical Equipment and Systems) and BS 7671 (a British Wiring Regulations standard).

Conforming to these standards can be challenging, especially when it is necessary to power high power imaging equipment. Consider, as an example, the power supply to imaging equipment such as Magnetic Resonance Imaging (MRI) machines, Computed Tomography (CT) machines and Angiography Machines. These devices can require in excess of 250 kVA to operate in full acquisition mode. However, to conform to the exacting power supply standards, the power supplies for this equipment must have a line to line impedance of less than 0.09 Ω at the point of supply and very low (or zero) earth leakage. To conform to these limits, the imaging equipment requires a separate electrical power supply to the main hospital *"low-risk"* power supply.

To complicate matters further, there is a requirement for medical imaging equipment to have a back-up power supply in case of a failure in the main power supply. Typically, hospitals will have a back-up generator to provide this back-up supply. However, these back up-power generators take approximately 15 seconds to start and come up to full power. In this 15 seconds, all of the imaging and accompanying computer equipment is likely to have crashed. Even once back-up power is available, most imaging equipment will take 10 to 15 minutes to restart. If such a power failure happens when a patient is being imaged, patient safety is compromised. When imaging is being used during the course of invasive procedures, such as for image guided surgery, such a power failure can be life threatening.

To remove this risk, an Uninterruptible Power Supply (UPS) may be used. UPSs are capable of supplying sufficient power to the medical imaging equipment until back-up power is available, avoiding any equipment failure. However, commercially available UPSs have severe limitations. Firstly, adding a typical UPS into the electrical circuit will alone add of the order of 0.10 Ω of electrical impedance. This means conforming to a line to line impedance of less than 0.09 Ω is not possible. In addition to failing to meet electrical standards, these high impedances will have a significant impact on the imaging quality of any connected imaging device. Finally, a UPS which is rated at 250 kVA will have a very large footprint approaching the same order as the footprint of the medical imaging suite itself. Consequently installing a UPS of this size in a hospital would severely impact the provision of other patient services in the hospital.

Therefore, there exists a need to provide improved systems and methods for supplying continuous power in specialist electrical safety environments.

### Summary of the Invention

According to a first aspect of the invention there is provided a switching panel for providing an uninterrupted power supply to a first power demand device, such as a medical imaging device, the switching panel comprising:
a first power supply input arranged to receive power from a first power supply at a first power rating;
a second power supply input arranged to receive power from a second power supply at a second power rating lower than the first power rating; and
a first power output to which power is supplied from either the first power supply input or from the second power supply input, to provide power to a said first power demand device;
wherein, the switching panel is arranged:
   in a first configuration to supply power from the first power supply input to the first power output;
   monitor if power received at the first power supply input has decreased below a first threshold;
   upon detecting a decrease in the power received at the first power supply input to a level below the first threshold, generate a signal to be sent to said first power demand device that power will be switched from the first power supply input to the second power supply input; and
   supply power in a second configuration to the first power output from the second power supply input.

The switching panel may be further arranged to:
monitor if power received at the first power supply input has increased above a second threshold;
upon detecting an increase in the power received at the first power supply input above the second threshold, generate a signal to be sent to the first power demand device that power will be switched from the second power supply input to the first power supply input; and
supply power from the first power supply input to the first power demand output.

The switching panel may be further arranged to:
emit an audible and/or visual alert when the switching panel detects that the power available from the first power supply input has increased above the second threshold; and,
send the signal which causes the switching panel to operate in its first configuration in response to receiving a user input.

The switching panel may further comprise a second power output, the second power output being operably connectable to a second power demand device.

The switching panel may be further arranged to:
supply power to the second power output from the second power supply input; and
upon detecting a decrease in the power received at the first power supply input, continuing to supply power from the second power supply input to the second power output.

The second power demand device may require less power than the first power demand device in the first power mode.

The first power supply may be a mains power supply, a three-phase power supply, or a direct-current power supply. The second power supply may be a persistent power supply. The persistent power supply may comprise one or more uninterruptible power supplies or batteries.

If the first power demand device is a medical imaging device, when the switching panel operates in its first configuration the first power demand device may operate in a full imaging mode; and when the switching panel operates in its second configuration the first power demand device may operate in a partial imaging mode.

The medical imaging device may be an angiography machine, the full imaging mode may be an acquisition mode and the partial imaging mode may be a fluoroscopy mode.

The switching panel may further comprise a bypass switch, the bypass switch may be operable to prevent the switching panel from supplying power from the first power supply.

The bypass switch may be further arranged to be operable to prevent the switching panel from supplying power from the second power supply.

The first mode of the first demand device may be a high power mode, wherein the second mode of the first demand device may be a low power mode, wherein the signal which causes the first power demand device to switch into the second power mode may cause the first power demand device to switch into the low power mode from the high power mode.

According to a second aspect of the invention there is provided a system comprising:
a switching panel;
a first power supply arranged to supply power to the switching panel, for example, at a first power rating;
a second power supply arranged to supply power to the switching panel, for example, at a second power rating lower than the first power rating; and
a first power demand device, such as a medical imaging device, the power demand device having a first power mode and a second power mode,
wherein, the switching panel is arranged to:
supply power to the first power demand device from either the first power supply or the second power supply;
monitor the power received from the first power supply; and
upon detecting a decrease in the power received from the first power supply,
send a signal which causes the first power demand device to switch into the
second power mode and supply power to the first power demand device from
the second power supply.

The switching panel may be further arranged to:
detect when the power available from the first power supply has increased above a threshold; and
send a signal which causes the power demand device to switch into the first power mode, and supply power to the power demand device from the first power supply.

The switching panel may be further arranged to:
emit an audible and/or visual alert when the switching panel detects that the power available from the first power supply has increased above the threshold;
and, send the signal which causes the power demand device to switch into the first power mode in response to receiving a user input.

The system may further comprise:
a second power demand device, and
wherein the switching panel is further arranged to supply power to the second power demand device from the second power supply.

The second power demand device may require less power than the first power demand device in the first power mode.

The first power supply may be a mains power supply, a three-phase power supply, or a direct-current power supply. The second power supply may be a persistent power supply. The persistent power supply may comprise one or more uninterruptible power supplies or batteries.

If the first demand device is a medical imaging device, the first power mode may be a full imaging mode and the second power mode may be a partial imaging mode.

The medical imaging device may be an angiography machine, the full imaging mode may be an acquisition mode and the partial imaging mode may be a fluoroscopy mode.

The switching panel may further comprise a bypass switch, the bypass switch being operable to prevent the switching panel from supplying power from the first power supply.

The bypass switch may be further arranged to be operable to prevent the switching panel from supplying power from the second power supply.

According to a further aspect of the invention there is provided a method of supplying power to a first power demand device, such as a medical imaging device, having a first power mode and a second power mode, the method comprising the steps of:
supplying power to the first power demand device from a first power supply;
monitoring the power received from the first power supply; and
upon detecting a decrease in the power received from the first power supply,
sending a signal which causes the first power demand device to switch into the second power mode, and
supplying power to the first power demand device from a second power supply.

The method may further comprise the steps of:
detecting when the power available from the first power supply has increased above a threshold;
sending a signal which causes the first power demand device to switch into the first power mode; and
supplying power to the first power demand device from the first power supply.

The method may further comprise the steps of:
emitting an audible and/or visual alert when it is detected that the power available from the first power supply has increased above the threshold; and,
sending the signal which causes the first power demand device to switch into the first power mode in response to receiving a user input.

The method may further comprise the steps of:
supplying power to a second power demand device from the second power supply; and
upon detecting a decrease in the power received from the first power supply, continuing to supply power to the second power demand device from the second power supply.

The first power supply may be a mains power supply, a three-phase power supply, or a direct-current power supply. The second power supply may be a persistent power supply. The persistent power supply may comprise one or more uninterruptible power supplies (UPSs) or batteries.

If the first demand device is a medical imaging device, the first power mode may be a full imaging mode and the second power mode may be a partial imaging mode.

The medical imaging device may be an angiography machine, the full imaging mode may be an acquisition mode and the partial imaging mode may be a fluoroscopy mode.

The first mode of the first demand device may be a high power mode, the second mode of the first demand device may be a low power mode, and the signal which causes the first power demand device to switch into the second power mode may cause the first power demand device to switch into the low power mode from the high power mode.

According to a further aspect of the invention there is provided a medical imaging suite comprising a switching panel as described above.

### Brief Description of the Drawings

By way of example only, the present invention will now be described with reference to the drawings, in which:
Figure 1 shows a schematic diagram of a power supply system in accordance with a first embodiment of the invention;
Figure 2 shows a method of operating the power supply system of Figure 1;
Figure 3 shows a schematic diagram of a first configuration of a power supply system in accordance with a second embodiment of the invention;
Figure 4 shows the power supply system of Figure 3 in a second configuration; and
Figure 5 shows a method of operating the power supply system of Figures 3 and 4.

### Detailed Description of Preferred Embodiments

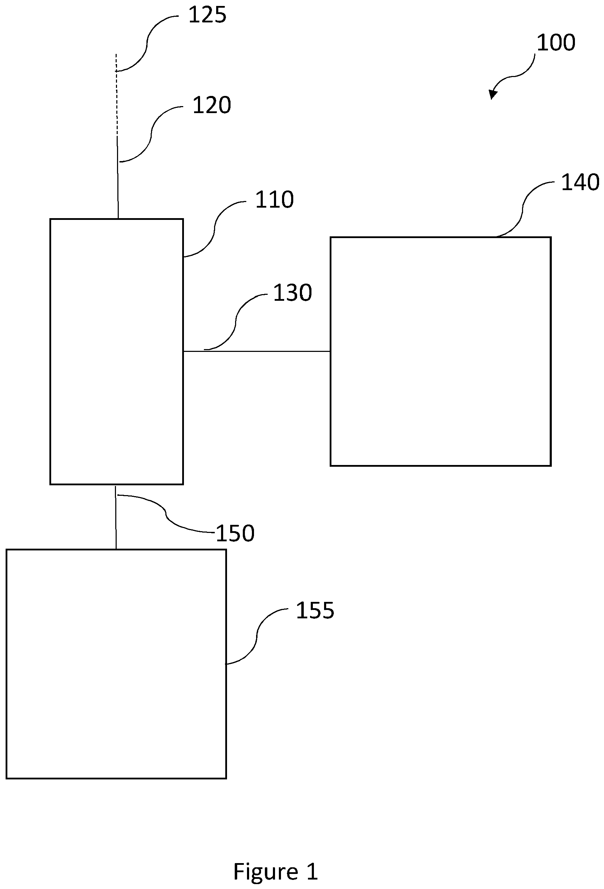

A first embodiment of the present invention will now be described with reference to Figure 1 which illustrates a power supply system 100.

The power supply system 100 comprises a switching panel 110. The switching panel 110 comprises a first power supply input 120 arranged to receive power from a first power supply 125. The first power supply 125 may be any power supply such as a mains power supply, a three-phase power supply, or a direct-current power supply. The first power supply 125 is, under normal operating conditions, the main power supply used to power any connected equipment.

The switching panel 110 also comprises at least one output 130. The output 130 is arranged to output power to any connected device. In the present example, the output 130 is shown as being in electrical connection with a power demand device 140. The power demand device 140 may be any device which consumes electrical power and that has at least two power modes, namely a low power mode and a high power mode. The power demand device may be a medical imaging device where the high power mode is a full imaging mode and the low power mode is a partial imaging mode. For example, the medical imaging device may be an angiography machine, the full imaging mode may be an acquisition mode and the partial imaging mode may be a fluoroscopy-only mode.

The switching panel 110 also comprises a second power supply input 150 arranged to receive power from a second power supply 155. The second power supply is a persistent power supply. I.e. a power supply that is able to instantly provide power, for a period of time, in case of interruption of the first power supply 125. The persistent power supply may be one or more uninterruptible power supplies (UPSs) or batteries.

The switching panel 110 is also configured to monitor the status of the first power supply 125. Thus the switching panel 110 also comprises a further module (not shown) arranged to monitor the first power supply 125 and detect any reduction in power received from the first power supply 125. The method of monitoring the status of the first power supply 125 is not important to the understanding of the invention. Any suitable monitoring means may be used, such as a volt and/or current meter.

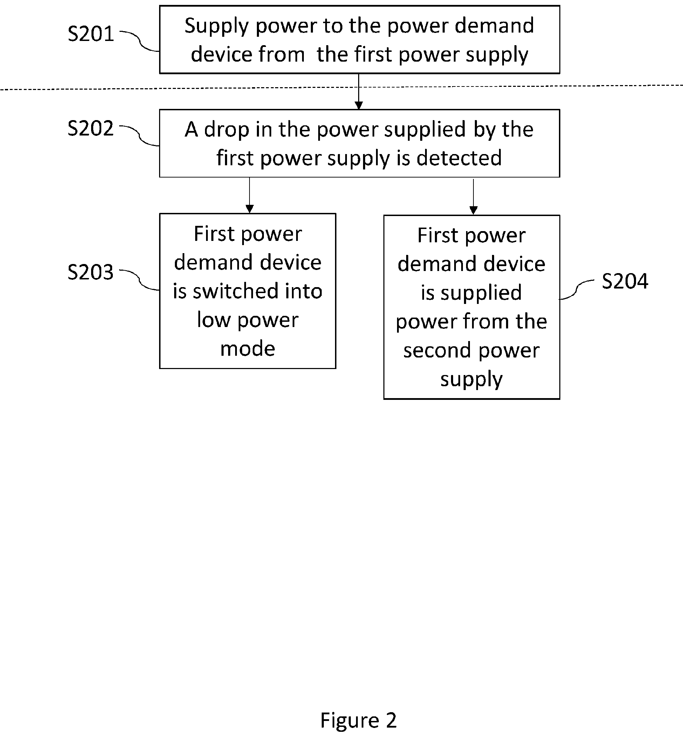

The operation of the power supply system 100 will now be described in more detail, with reference to Figure 2. Figure 2 is a flow-chart showing the operation of the power supply system 100.

Under normal operating conditions, as shown at step S201, the switching panel 110 is configured in a first configuration to supply power to the power demand device 140 from the first power supply 125. At step S202, the switching panel 110 detects a drop in the power supplied by the first power supply. Following this detection, the switching panel 110 performs two further steps, S203 and S204.

At step S203, the switching panel 110 sends a signal to the first power demand device 140, which forces the first power demand device to switch into a low power mode. This signal may be any signal suitable to effect this switch. For example, the switching panel 110 may further comprise a communications module (not shown) which is operable to send a suitable signal to the first power demand device. The communications module may use any suitable wired or wireless communication technology. In some implementation of the invention, it may be possible to use features of the first power demand device to implement this signalling process. For example, in the case of hospital imaging suites, many ionising radiation imaging machines are configured to immediately switch into a low power (non-imaging) mode upon detection of movement of a door (to prevent unwanted ionising radiation exposure); in this case, the control signal may comprise the same signal used to indicate movement of the door.

At step S204, the switching panel 110 switches to a second configuration, in which the switching panel 110 supplies power to the first power demand device 140 from the second power supply 155. The second power supply 155 is able to provide sufficient power to power the first power demand device 140 until either resumption of the supply from the first power supply 125 or back-up generator power is available.

Figure 2 illustrates steps S203 and S204 occurring at the same time. Alternatively, steps S203 and S204 could occur in short succession, as long as the power demand device 140 does not experience an interruption in its power supply sufficient to disrupt its operation.

One advantage of the present invention is that it enables use of smaller, lower rated UPSs to provide an emergency persistent power supply to bridge the gap between failure of the main power supply and the availability of generated back-up power. This has significant efficiency advantages in that a lower rated UPS includes less batteries than a higher rated UPS, thereby requiring less electricity to maintain the batteries' charge and requiring less maintenance. In addition, when the working life of the UPS is reached and the batteries are decommissioned there will be less environmentally damaging material to be disposed of.

The present invention relies on the use of commonly available low-power modes, to avoid overloading the UPS. A further advantage of the presently claimed invention is that suitable smaller persistent power supplies (such as 40 kVA UPSs) are available with impedance ratings low enough (sub 0.09 Ω) to avoid affecting the power demand device 140. Moreover, an advantage of providing the second power supply 155 in an electrically isolatable circuit from the first power supply 125, is that the second power supply 155 does not contribute to the impedance of the first power supply 125. Thus the present invention provides an improved power supply arrangement which is suitable for use in specialised electrical safety environments.

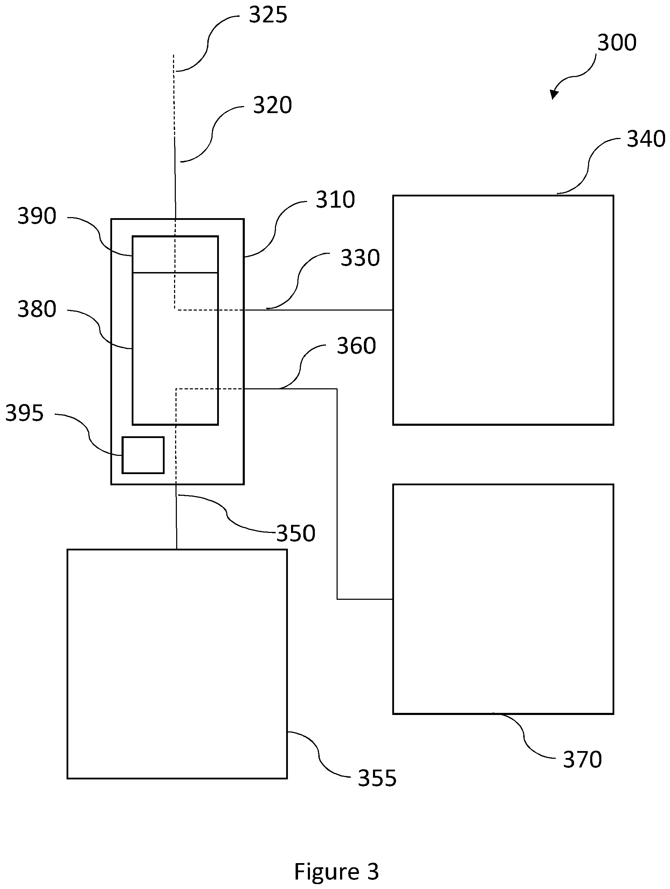

A further embodiment of the invention will now be described with reference to Figure 3 which illustrates a power supply system 300. The power supply system 300 comprises all of the features of the first embodiment of the invention. That is, the power supply system 300 comprises a switching panel 310.

The switching panel 310 comprises a first power supply input 320 arranged to receive power from a first power supply 325. The switching panel 310 also comprises a first power output 330. In the present example, the output 330 is shown as being in electrical connection with a first power demand device 340. The first power demand device 340 may be any device which consumes electrical power and that has at least two power modes, namely a low power mode and a high power mode.

The switching panel 310 also comprises a second power supply input 350 arranged to receive power from a second power supply 355. The second power supply is a persistent power supply. The switching panel 310 is also configured to monitor the status of the first power supply 325. Thus the switching panel 310 also comprises a further module (not shown) arranged to monitor the first power supply 325 and detect any reduction in power received from the first power supply 325.

In the present embodiment, the power supply system 300 further comprises a second power output 360. The second power output 360 is configured to output power to any connected device. In the present example, the second power output 360 is shown as being in electrical connection with a second power demand device 370.

The second power demand device 370 may be any device which consumes electrical power. However, in embodiments of the invention where the second power supply 355 is capable of supplying less power than the first power supply 325, the second power demand device 370 must demand less power than the first power demand device 340. The second power demand device 370 may be ancillary, non-safety critical, electrical equipment required to run and/or support the first power demand device 340. For example, where the first power demand device 340 is a medical imaging device, the second power demand device 370 may be one or more control computers, recording devices, non-medical electrical devices and so on.

In this embodiment, the switching panel 310 is arranged in its first configuration (corresponding to normal operating conditions), to supply power to the first power demand device 340 from the first power supply 325; and to supply power to the second power demand device 370 from the second power supply 355. To illustrate these electrical connections, switching panel 310 has been illustrated as comprising a switching unit 380, which shows the interior electrical connections of the switching panel 310 with dashed lines.

The switching panel 310 also comprises a bypass switch 390. The bypass switch 390 is operably connected to the switching unit 380. The bypass switch 390 is operable to selectively switch the switching panel between receiving power from the first power supply 325 and/or the second power supply 355. The bypass switch may be manually operable, allowing a user to isolate one or more of the first and second power supplies.

The switching panel 310 also comprises an alarm unit 395. The alarm unit 395 is configured to emit an audible and/or visual alert when the switching panel 310 detects that the power available from the first power supply 325 has decreased. The alarm unit 395 thus alerts anyone within range of the audible and/or visual alert to a reduction in power and the subsequent switching of the first power demand device 340 to its lower power mode.

The alarm unit 395 may be further configured to emit an audible and/or visual alert when the switching panel detects that the power available from the first power supply 325 has increased above a threshold. The threshold is a power level at which the first power demand device may run, in either low power or high power mode.

The switching panel 310 may be further configured to delay sending a signal which causes the first power demand device 340 to switch back into the first power mode until it has received a user input. The user input may be manual operation of the bypass switch 390. Alternatively, the user input may be any suitable input which indicates to the switching panel 310 a user acknowledgement, such as a button push.

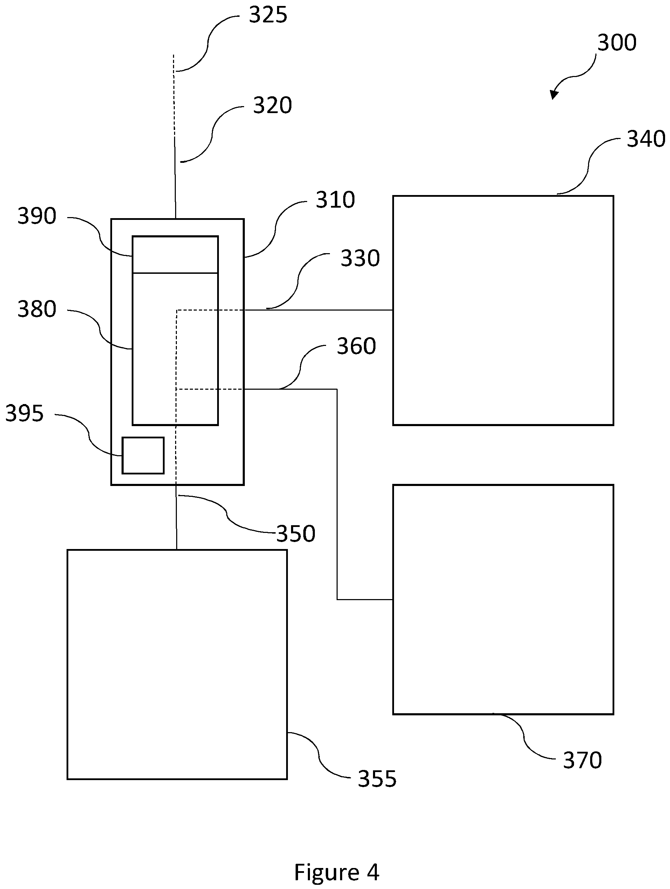

Figure 4 shows the same power supply system 300 of Figure 3. However, Figure 4 illustrates how the switching panel 310 is arranged after detection of a drop in the power supplied by the first power supply 325. Dashed lines in the switching panel 310, and the switching unit 380, illustrate that the second power supply 355 supplies power to both the first power demand device 340 and to the second power demand device 370.

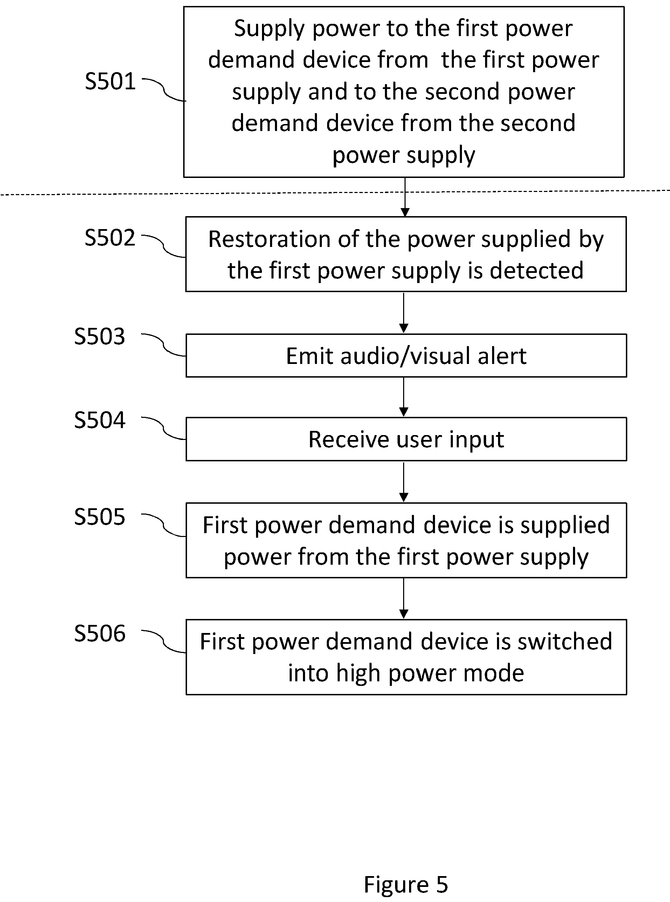

The operation of the power supply system 300 will now be described in more detail, with reference to Figure 5. Figure 5 is a flow-chart showing the operation of the power supply system 300 upon restoration of the power available from the first power supply 325.

At step S501, the switching panel 310 is configured in the second configuration, a "switched" arrangement, supplying power to both the first power demand device 340 and the second power demand device 370 from the second power supply 355, as shown in Figure 4. At step S502, the switching panel 310 detects that the power supplied by the first power supply 325 has been restored to a suitable level to resume powering the first power demand device 340.

At step S503, the switching panel 310 emits an audio and/or visual alert. The switching panel 310 then waits to receive a user input. At step S504, the switching panel 310 receives this input from a user. At step S505, the switching panel 310 supplies power to the first power demand device 340 from the first power supply 325. The switching panel 310 does not alter the power supply to the second power demand device 370.

At step S506, the first power demand device 340 is switched back into its high power mode. To achieve this, the switching panel 310 sends a signal to the first power demand device 340, which forces the first power demand device 340 to switch into its high power mode. This signal may be any signal suitable to effect this switch. The switching panel 310 may send this signal at the same time, or shortly after, the first power demand device 340 is supplied power from the first power supply 325 (step S505). Alternatively, the switching panel 310 may require a further user input before sending this signal.

The above method is described in conjunction with the second embodiment of the invention for illustrative purposes only. It should be understood that the above described method applies equally to the first embodiment of the invention (where the second power demand device 370 is not considered).

Features of the present invention are defined in the appended claims.

The above embodiments describe one way of implementing the present invention. It will be appreciated that modifications of the features of the above embodiments are possible within the scope of the independent claims.

### CLAUSES

There follows a list of numbered clauses defining particular embodiments of the invention. Where a numbered clause refers to an earlier numbered clause then those clauses may be considered in combination.
1. A switching panel for providing an uninterrupted power supply to a first power demand device, the switching panel comprising:
   a first power supply input arranged to receive power from a first power supply at a first power rating;
   a second power supply input arranged to receive power from a second power supply at a second power rating lower than the first power rating; and
   a first power output to which power is supplied from either the first power supply input or from the second power supply input, to provide power to a said first power demand device;
   wherein, the switching panel is arranged:
      in a first configuration to supply power from the first power supply input to the first power output;
      monitor if power received at the first power supply input has decreased below a first threshold;
      upon detecting a decrease in the power received at the first power supply input to a level below the first threshold, generate a signal to be sent to said first power demand device that power will be switched from the first power supply input to the second power supply input; and
      supply power in a second configuration to the first power output from the second power supply input.
2. The switching panel of clause 1, wherein the switching panel is further arranged to:
   monitor if power received at the first power supply input has increased above a second threshold;
   upon detecting an increase in the power received at the first power supply input above the second threshold, generate a signal to be sent to said first power demand device that power will be switched from the second power supply input to the first power supply input; and
   supply power from the first power supply input to the first power demand output.
3. The switching panel of clause 2, wherein the switching panel is further arranged to:
   emit an audible and/or visual alert when the switching panel detects that the power available from the first power supply input has increased above the second threshold; and,
   send the signal which causes the switching panel to operate in its first configuration in response to receiving a user input.
4. The switching panel of any preceding clause, further comprising a second power output, the second power output being operably connectable to a second power demand device.
5. The switching panel of clause 4, wherein the switching panel is further arranged to:
   supply power to said second power output from said second power supply input; and
   upon detecting a decrease in the power received at the first power supply input, continuing to supply power from the second power supply input to the second power output.
6. The switching panel of either clause 4 or 5, wherein said second power demand device requires less power than said first power demand device in said first power mode.
7. The switching panel of any preceding clause, wherein the first power supply is a mains power supply, a three-phase power supply, or a direct-current power supply.
8. The switching panel of any preceding clause, wherein the second power supply is a persistent power supply.
9. The switching panel of clause 8, wherein the persistent power supply comprises one or more uninterruptible power supplies or batteries.
10. The switching panel of any preceding clause, wherein said first power demand device is a medical imaging device and when the switching panel operates in its first configuration said first power demand device operates in a full imaging mode; and when the switching panel operates in its second configuration said first power demand device operates in a partial imaging mode.
11. The switching panel of clause 10, wherein said medical imaging device is an angiography machine, said full imaging mode is an acquisition mode and said partial imaging mode is a fluoroscopy mode.
12. The switching panel of any preceding clause, further comprising a bypass switch, the bypass switch being operable to prevent the switching panel from supplying power from the first power supply.
13. The switching panel of any preceding clause, wherein the bypass switch is further arranged to be operable to prevent the switching panel from supplying power from the second power supply.
14. The switching panel of any preceding clause, wherein the first mode of said first demand device is a high power mode, wherein the second mode of said first demand device is a low power mode, wherein the signal which causes said first power demand device to switch into said second power mode causes said first power demand device to switch into said low power mode from said high power mode.
15. A system comprising:
   a switching panel;
   a first power supply arranged to supply power to the switching panel;
   a second power supply arranged to supply power to the switching panel; and
   a first power demand device, said power demand device having a first power mode and a second power mode,
   wherein, the switching panel is arranged to:
   supply power to said first power demand device from either the first power supply or the second power supply;
   monitor the power received from the first power supply; and
   upon detecting a decrease in the power received from the first power supply,
   send a signal which causes said first power demand device to switch into said
   second power mode and supply power to said first power demand device from
   said second power supply.
16. The system of clause 15, wherein the switching panel is further arranged to:
   detect when the power available from the first power supply has increased above a threshold; and
   send a signal which causes said power demand device to switch into said first power mode, and supply power to said power demand device from said first power supply.
17. The system of clause 16, wherein the switching panel is further arranged to:
   emit an audible and/or visual alert when the switching panel detects that the power available from the first power supply has increased above the threshold;
   and, send the signal which causes said power demand device to switch into said first power mode in response to receiving a user input.
18. The system of any of clauses 15 to 17, further comprising:
   a second power demand device, and
   wherein the switching panel is further arranged to supply power to said second power demand device from the second power supply.
19. The system of clause 18, wherein the second power demand device requires less power than the first power demand device in said first power mode.
20. The system of any of clauses 15 to 19, wherein the first power supply is a mains power supply, a three-phase power supply, or a direct-current power supply.
21. The system of any of clauses 15 to 20, wherein the second power supply is a persistent power supply.
22. The system of clause 21, wherein the persistent power supply comprises one or more uninterruptible power supplies or batteries.
23. The system of any of clauses 15 to 19, wherein said first demand device is a medical imaging device, said first power mode is a full imaging mode and said second power mode is a partial imaging mode.
24. The system of clause 23, wherein said medical imaging device is an angiography machine, said full imaging mode is an acquisition mode and said partial imaging mode is a fluoroscopy mode.
25. The system of any of clauses 15 to 24, wherein the switching panel further comprises a bypass switch, the bypass switch being operable to prevent the switching panel from supplying power from the first power supply.
26. The system of clause 25, wherein the bypass switch is further arranged to be operable to prevent the switching panel from supplying power from the second power supply.
27. A method of supplying power to a first power demand device having a first power mode and a second power mode, the method comprising the steps of:
   supplying power to the first power demand device from a first power supply;
   monitoring the power received from the first power supply; and
   upon detecting a decrease in the power received from the first power supply,
   sending a signal which causes said first power demand device to switch into
   said second power mode, and
   supplying power to said first power demand device from a second power
      supply.
28. The method of clause 27, further comprising the steps of:
   detecting when the power available from the first power supply has increased above a threshold;
   sending a signal which causes said first power demand device to switch into said first power mode; and
   supplying power to said first power demand device from said first power supply.
29. The method of clause 28, further comprising the steps of:
   emitting an audible and/or visual alert when it is detected that the power available from the first power supply has increased above the threshold; and,
   sending the signal which causes said first power demand device to switch into said first power mode in response to receiving a user input.
30. The method of any of clauses 27 to 29, further comprising the steps of:
   supplying power to a second power demand device from said second power supply; and
   upon detecting a decrease in the power received from the first power supply, continuing to supply power to said second power demand device from said second power supply.
31. The method of any of clauses 27 to 30, wherein the first power supply is a mains power supply, a three-phase power supply, or a direct-current power supply.
32. The method of any of clauses 27 to 31, wherein the second power supply is a persistent power supply.
33. The method of clause 32, wherein the persistent power supply comprises one or more uninterruptible power supplies, UPSs, or batteries.
34. The method of any of clauses 27 to 33, wherein said first demand device is a medical imaging device, said first power mode is a full imaging mode and said second power mode is a partial imaging mode.
35. The method of any clause 34, wherein said medical imaging device is an angiography machine, said full imaging mode is an acquisition mode and said partial imaging mode is a fluoroscopy mode.
36. The method of any of clauses 27 to 35, wherein the first mode of said first demand device is a high power mode, wherein the second mode of said first demand device is a low power mode, wherein the signal which causes said first power demand device to switch into said second power mode causes said first power demand device to switch into said low power mode from said high power mode.
37. A medical imaging suite comprising a switching panel in accordance with any of clauses 1 to 14.
38. A medical imaging suite comprising a system in accordance with any of clauses 15 to 26.

## Claims

1. A switching panel for providing an uninterrupted power supply to a medical imaging device, the switching panel comprising:
a first power supply input arranged to receive power from a first power supply at a first power rating;
a second power supply input arranged to receive power from a second power supply at a second power rating lower than the first power rating; and
a first power output to which power is supplied from either the first power supply input or from the second power supply input, to provide power to a said medical imaging device;
wherein, the switching panel is arranged:
in a first configuration to supply power from the first power supply input to the first power output;
monitor if power received at the first power supply input has decreased below a first threshold;
upon detecting a decrease in the power received at the first power supply input to a level below the first threshold, generate a signal to be sent to said medical imaging device that power will be switched from the first power supply input to the second power supply input; and
supply power in a second configuration to the first power output from the second power supply input; and
wherein when the switching panel operates in the first configuration said medical imaging device can operate in a full imaging mode and when the switching panel operates in its second configuration said medical imaging device can operate in a partial imaging mode.

2. The switching panel of claim 1, wherein the switching panel is further arranged to:
monitor if power received at the first power supply input has increased above a second threshold;
upon detecting an increase in the power received at the first power supply input above the second threshold, generate a signal to be sent to said medical imaging device that power will be switched from the second power supply input to the first power supply input; and
supply power from the first power supply input to the first power output.

3. The switching panel of any preceding claim, further comprising a second power output, the second power output being operably connectable to a second power demand device, wherein the switching panel is further arranged to:
supply power to said second power output from said second power supply input; and
upon detecting a decrease in the power received at the first power supply input, continuing to supply power from the second power supply input to the second power output, wherein said second power demand device requires less power than said first power demand device in said first power mode.

4. The switching panel of any preceding claim, wherein the first power supply is a mains power supply, a three-phase power supply, or a direct-current power supply, wherein the second power supply is a persistent power supply, and wherein the persistent power supply comprises one or more uninterruptible power supplies or batteries.

5. The switching panel of any preceding claim, further comprising a bypass switch, the bypass switch being operable to prevent the switching panel from supplying power from the first power supply, wherein the bypass switch is further arranged to be operable to prevent the switching panel from supplying power from the second power supply.

6. A system comprising:
a switching panel;
a first power supply arranged to supply power to the switching panel at a first power rating;
a second power supply arranged to supply power to the switching panel at a second power rating lower than the first power rating; and
a medical imaging device, said medical imaging device having a first power mode and a second power mode, wherein said first power mode is a full imaging mode and said second power mode is a partial imaging mode,
wherein the switching panel is arranged to:
supply power to said medical imaging device from either the first power supply or the second power supply;
monitor the power received from the first power supply; and
upon detecting a decrease in the power received from the first power supply, send a signal which causes said medical imaging device to switch into said second power mode and supply power to said medical imaging device from said second power supply.

7. The system of claim 6, wherein the switching panel is further arranged to:
detect when the power available from the first power supply has increased above a threshold; and
send a signal which causes said medical imaging device to switch into said first power mode, and supply power to said medical imaging device from said first power supply.

8. The system of claim 6 or 7, further comprising:
a second power demand device, and
wherein the switching panel is further arranged to supply power to said second power demand device from the second power supply, wherein the second power demand device requires less power than the medical imaging device in said first power mode.

9. The system of any of claims 6 to 8, wherein the first power supply is a mains power supply, a three-phase power supply, or a direct-current power supply, wherein the second power supply is a persistent power supply, and wherein the persistent power supply comprises one or more uninterruptible power supplies or batteries.

10. The system of any of claims 6 to 9, wherein the switching panel further comprises a bypass switch, the bypass switch being operable to prevent the switching panel from supplying power from the first power supply, wherein the bypass switch is further arranged to be operable to prevent the switching panel from supplying power from the second power supply.

11. A method of supplying power to a medical imaging device having a first power mode and a second power mode, wherein said first power mode is a full imaging mode and said second power mode is a partial imaging mode, the method comprising the steps of:
supplying power to the medical imaging device from a first power supply at a first power rating;
monitoring the power received from the first power supply;
upon detecting a decrease in the power received from the first power supply,
sending a signal which causes said medical imaging device to switch into said second power mode, and
supplying power to said medical imaging device from a second power supply at a second power rating lower than the first power rating.

12. The method of claim 11, further comprising the steps of:
detecting when the power available from the first power supply has increased above a threshold;
sending a signal which causes said medical imaging device to switch into said first power mode; and
supplying power to said medical imaging device from said first power supply.

13. The method of any of claims 11 to 12, further comprising the steps of:
supplying power to a second power demand device from said second power supply; and
upon detecting a decrease in the power received from the first power supply, continuing to supply power to said second power demand device from said second power supply.

14. The method of any of claims 11 to 13, wherein the first power supply is a mains power supply, a three-phase power supply, or a direct-current power supply, wherein the second power supply is a persistent power supply, and wherein the persistent power supply comprises one or more uninterruptible power supplies, UPSs, or batteries.

15. A medical imaging suite comprising a switching panel in accordance with any of claims 1 to 6, or a system in accordance with any of claims 7 to 10.
